# FASCICULE DE BREVET EUROPEEN

(11) **EP 0 847 749 B1**
(45) Date de publication et mention de la délivrance du brevet: **01.12.1999**
(21) Numéro de dépôt: 97402780.7
(22) Date de dépôt: 19.11.1997
(51) Int. Cl.: A61K 7/13

(54) **Utilisation de dérivés de la Di-imino-isoindoline ou de la 3-amino-isoindolone pour la teinture des fibres kératiniques et compositions de teinture les renfermant**
Verwendung von Diimino-isoindolin- oder 3-Amino-isoindolon-Derivaten zum Färben von Keratinfasern und Zusammensetzungen zum färben, die diese enthalten
Use of diiminoisoindoline or 3-aminoisoindolone derivatives for the dyeing of keratinic fibers and compositions containing them

(30) Priorité: 12.12.1996 FR 9615290
(43) Date de publication de la demande: 17.06.1998
(73) Titulaire: L'OREAL, 75008 Paris (FR)
(72) Inventeur: Andrean, Hervé, 75014 Paris (FR); Lagrange, Alain, 77700 Coupvray (FR)
(74) Mandataire: Miszputen, Laurent

(56) Documents cités:
- DE-A- 4 335 623
- DE-A- 4 409 143
- GB-A- 1 020 305
- GB-A- 2 181 750
- DATABASE WPI Week 8306 Derwent Publications Ltd., London, GB; AN 83-13364k XP002039556 & JP 57 210 077 A (SUMITOMO CHEM CO LTD)

## Description

La présente invention concerne l'utilisation de dérivés de la di-imino-isoindoline ou de la 3-amino-isoindolone ou de leurs formes tautomères pour la teinture des fibres kératiniques, en particulier pour fibres kératiniques humaines telles que les cheveux, les compositions de teinture les renfermant et les procédés et dispositifs de teinture mettant en oeuvre ces compositions.
Pour la teinture des fibres kératiniques, et en particulier les cheveux, il est connu d'utiliser des colorants directs ou substances colorées qui confèrent à la fibre une coloration temporaire ou semi-permanente, de faible puissance tinctoriale et qui s'élimine généralement aux lavages ou à la transpiration, ou des colorants d'oxydation (bases d'oxydation et coupleurs) qui sont des composés initialement incolores ou faiblement colorés, engendrant sous l'action d'un oxydant, des composés colorés par un processus de condensation oxydative. Les colorations d'oxydation sont, comparativement aux colorations directes, permanentes, puissantes, et résistantes aux agents extérieurs (lumière, intempéries, lavages, transpiration et frottements).
On sait également teindre les fibres kératiniques avec une association de dérivés d'isatine N-substitués et de composés aminofonctionnels comme il a été décrit dans la demande de brevet DE 4409143, ou encore sans oxydant en présence d'une association de dérivés d'indolinone et en particulier la 3-imino-2-indolinone et d'une amine primaire ou secondaire tel qu'il a été décrit dans la demande DE 4335623.
La demanderesse a maintenant découvert, de façon totalement inattendue et surprenante, que des dérivés de la di-imino-isoindoline ou de la 3-amino-isoindolone ou leurs formes tautomères de formule (I) définie ci-après, qui sont des molécules incolores, pouvaient teindre les fibres kératiniques en l'absence d'agent oxydant, par réaction avec certains réactifs spécifiques, en particulier des composés à fonction amine primaire ou secondaire.

Cette découverte est à la base de la présente invention.
La présente invention a ainsi pour objet l'utilisation de dérivés de la di-imino-isoindoline ou de la 3-amino-isoindolone ou de leurs formes tautomères de formule (I) définie ci-après, pour la teinture des fibres kératiniques, en particulier des fibres kératiniques humaines telles que les cheveux.

Elle a aussi pour objet l'utilisation des dérivés susnommés, à titre de précurseurs de coloration sans oxydant, dans des, ou pour la préparation de, compositions de teinture des fibres kératiniques, en particulier des fibres kératiniques humaines telles que les cheveux.

On entend par précurseur de coloration sans oxydant, un composé essentiellement incolore susceptible d'engendrer une coloration, sans l'apport d'un agent oxydant, par réaction avec un autre composé réagissant avec lui.

La présente invention a également pour objet l'utilisation des dérivés susnommés, à titre de précurseurs de coloration sans oxydant, en association avec un composé à fonction amine primaire ou secondaire, dans des compositions de teinture des fibres kératiniques, en particulier des fibres kératiniques humaines telles que les cheveux.

L'invention concerne en outre les compositions de teinture des fibres kératiniques, en particulier des fibres kératiniques humaines telles que les cheveux, contenant, dans un milieu approprié pour la teinture, au moins un dérivé de la di-imino-isoindoline ou de la 3-amino-isoindolone ou sa forme tautomère de formule (I) définie ci-après, à titre de précurseur de coloration sans oxydant.

L'invention concerne aussi les compositions de teinture des fibres kératiniques, en particulier des fibres kératiniques humaines telles que les cheveux, contenant, dans un milieu approprié pour la teinture, (i) au moins un dérivé de la di-imino-isoindoline ou de la 3-amino-isoindolone ou sa forme tautomère de formule (I) définie ci-après et (ii) un composé à fonction amine primaire ou secondaire.

L'invention a pour autre objet une composition à deux composants pour lesquels, dans un milieu approprié pour la teinture, l'un contient au moins un dérivé de la di-imino-isoindoline ou de la 3-amino-isoindolone ou sa forme tautomère de formule (I) définie ci-après, l'autre un composé à fonction amine primaire ou secondaire, et qui, stockés de façon séparée, sont (i) mélangés au moment de l'emploi pour l'application sur les fibres kératiniques ou (ii) appliqués séquentiellement sur lesdites fibres.

Elle a également pour objet les procédés de teinture mettant en oeuvre ces compositions.

Un autre objet de l'invention concerne un dispositif à plusieurs compartiments, ou «kits», pour la teinture des fibres kératiniques, caractérisé par le fait qu'il comporte au moins deux compartiments, dont l'un d'eux renferme une composition contenant, dans un milieu approprié pour la teinture, au moins un dérivé de la di-imino-isoindoline ou de la 3-amino-isoindolone ou sa forme tautomère de formule (I) définie ci-après et l'autre renferme une composition contenant, dans un milieu approprié pour la teinture, un composé susceptible de réagir sans oxydant avec le composé de formule (I) pour former un colorant.

L'invention concerne également un dispositif à plusieurs compartiments, ou «kits», pour la teinture des fibres kératiniques, caractérisé par le fait qu'il comporte au moins deux compartiments, dont l'un d'eux renferme une composition contenant, dans un milieu approprié pour la teinture, au moins un dérivé de la di-imino-isoindoline ou de la 3-amino-isoindolone ou sa forme tautomère de formule (I) définie ci-après et l'autre renferme une composition contenant, dans un milieu approprié pour la teinture, un composé à fonction amine primaire ou secondaire.

Les teintures obtenues avec les compositions selon l'invention sont par ailleurs puissantes et résistantes aux agents extérieurs (lumière, intempéries, lavages, transpiration et frottements).

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Les dérivés de la di-imino-isoindoline ou de la 3-amino-isoindolone ou de leurs formes tautomères sont des composés de formule (I) suivante : 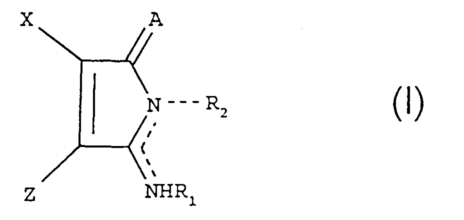 dans laquelle,
**R**_{**1**} et **R**_{**2**} désignent, indépendamment l'un de l'autre, H, alkyle, hydroxyalkyle, polyhydroxyalkyle, alkylhydroxyalkyle, aminoalkyle (l'amine pouvant être protégée par un radical acétyle, uréido, sulfonyle), alkylaminoalkyle, (dihydroxy)alkylaminoalkyle, ou alkyle-NR'R" (dans lequel R' et R" sont alkyle ou peuvent former ensemble avec l'atome d'azote auxquels ils sont rattachés, un cycle aliphatique ou hétérocyclique à 5 ou 6 chaînons),
   étant entendu que tous les radicaux alkyle des groupements ci-avant définis comportent de 1 à 4 atomes de carbone et peuvent être linéaires ou ramifiés, et que R₂ peut être nul,
**A** désigne O ou NH,
**X** et **Z** forment ensemble un cycle hydrocarboné à 5 ou 6 chaînons, saturé ou insaturé, aromatique ou hétérocyclique pouvant être interrompu par un ou plusieurs atomes d'azote ou de soufre, et qui peut être substitué par un ou plusieurs radicaux tels que NO₂, NH₂, acétylamino, OH, SO₃H, halogène (Br, Cl, F), CH₃SO₂, -CF₃, -OCF₃, alkyle en C₁-C₄, alcoxy(C₁-C₄), alkyl(C₁-C₄)thio, alcoxy (C₁-C₄)carbonyle,
et les sels cosmétiquement acceptables de ces composés.

De tels composés sont connus en tant que tels, ont été préparés dans l'art antérieur, et sont notamment les suivants :
- 3-imino-3H-isoindol-ylamine,
- 3-imino-4-méthyl-3H-isoindol-1-ylamine,
- 3-imino-4-terbutyl-3H-isoindol-1-ylamine,
- 3-imino-7-nitro-3H-isoindol-1-ylamine,
- 3-amino-1-imino-1H-isoindol-4-ol,
- 3-imino-7-isopropoxy-3H-isoindol-1-ylamine,
- 3-imino-7-(2,2,2-trifluoroéthoxy)-3H-isoindol-1-ylamine,
- 3-imino-7-éthoxy-3H-isoindol-1-ylamine,
- 3-imino-7-butoxy-3H-isoindol-1-ylamine,
- acide 3-amino-1-imino-1H-isoindole-4-sulfonique,
- 3-imino-7-chloro-3H-isoindol-1-ylamine,
- 3-imino-5-méthyl-3H-isoindol-1-ylamine,
- 3-imino-5-éthyl-3H-isoindol-1-ylamine,
- 3-imino-5-terbutyl-3H-isoindol-1-ylamine,
- 3-imino-5-amino-3H-isoindol-1-ylamine,
- N-(1-amino-3-imino-3H-isoindol-5-yl)-acétamide,
- 3-imino-5-nitro-3H-isoindol-1-ylamine,
- 3-imino-5-fluoro-3H-isoindol-1-ylamine,
- 3-imino-5-chloro-3H-isoindol-1-ylamine,
- 3-imino-5-méthylsulfanyl-3H-isoindol-1-ylamine,
- 3-imino-5-méthoxy-3H-isoindol-1-ylamine,
- 3-imino-5-éthoxy-3H-isoindol-1-ylamine,
- 3-imino-5-propoxy-3H-isoindol-1-ylamine,
- 3-imino-5-isopropoxy-3H-isoindol-1-ylamine,
- 3-imino-5-butoxy-3H-isoindol-1-ylamine,
- 3-imino-5-isobutoxy-3H-isoindol-1-ylamine,
- 3-imino-5-terbutoxy-3H-isoindol-1-ylamine,
- 3-imino-5-(2,2,2-trifluorométhyl)-3H-isoindol-1-ylamine,
- 3-imino-5-(2,2,2-trifluoroéthoxy)-3H-isoindol-1-ylamine,
- 3-imino-5-méthanesulfonyl-3H-isoindol-1-ylamine,
- 3-imino-5,6-diméthyl-3H-isoindol-1-ylamine,
- 3-imino-5,6-diéthyl-3H-isoindol-1-ylamine,
- 3-imino-5,6-diméthoxy-3H-isoindol-1-ylamine,
- 3-imino-5,6-diéthoxy-3H-isoindol-1-ylamine,
- 3-imino-5,6-dibutoxy-3H-isoindol-1-ylamine,
- 3-imino-5,6-bis-trifluorométhyl-3H-isoindol-1-ylamine,
- 3-imino-5,6-dichloro-3H-isoindol-1-ylamine,
- 5,6-bis-éthoxyméthyl-3-imino-3H-isoindol-1-ylamine,
- 3-amino-1-imino-1H-isoindole-4,7-diol,
- 4,7-dichloro-3-imino-3H-isoindol-1-ylamine,
- 4,5,7-trichloro-3-imino-N6,N6-diméthyl-3H-isoindole-1,6-diamine,
- 4,5,6,7-tétrachloro-3-imino-3H-isoindol-1-ylamine,
- 4,5,6,7-tétrafluoro-3-imino-3H-isoindol-1-ylamine,
- 3-butylimino-3H-isoindol-1-ylamine,
- 2-(3-amino-isoindol-1-ylidèneamino)-éthanol,
- 3-(3-amino-isoindol-1-ylidèneamino)-3-méthyl-pentane-1,5-diol,
- N-(3-amino- isoindol-1-ylidène)-guanidine,
- 7-imino-7H-pyrrolo[3,4-b]pyridin-5-ylamine,
- 7-imino-7H-pyrrolo[3,4-b]pyrazin-5-ylamine,
- 7-imino-2,3-diméthyl-7H-pyrrolo[3,4-b]pyrazin-5-ylamine,
- 7-imino-7H-[1,4]dithiino[2,3-c]pyrrol-5-ylamine,
- 7-imino-2,3-diméthyl-7H-[1,4]dithiino[2,3-c]pyrrol-5-ylamine,
- 7-imino-2,3-dihydro-7H-[1,4]dithiino[2,3-c]pyrrol-5-ylamine,
- 7-imino-2-méthyl-2,3-dihydro-7H-[1,4]dithiino[2,3-c]pyrrol-5-ylamine,
- 3-amino-isoindol-1-one,
- 3-amino-7-méthyl-isoindol-1-one,
- 3-amino-7-hydroxyméthyl-isoindol-1-one,
- 3-amino-7-chloro-isoindol-1-one,
- 3-amino-4-chloro-isoindol-1-one,
- acide 3-amino-1-oxo-1H-isoindole-4-sulfonique,
- 3-amino-4-nitro-isoindol-1-one,
- 3-amino-6-nitro-isoindol-1-one,
- 3-amino-6-méthyl-isoindol-1-one,
- 3-amino-6-chloro-isoindol-1-one,
- 3-amino-6-bromo-isoindol-1-one,
- 3-amino-6-méthylsulfanyl-isoindol-1-one,
- 3-amino-6-méthoxy-isoindol-1-one,
- 3-amino-5-chloro-isoindol-1-one,
- 3-amino-5-fluoro-isoindol-1-one,
- 3-amino-5-méthoxy-isoindol-1-one,
- 3-amino-5-nitro-isoindol-1-one,
- ester éthylique de l'acide 3-amino-1-oxo-1H-isoindole-5-carboxylique,
- 3-amino-5,6-dichloro-isoindol-1-one,
- 3-amino-5,6-dibromo-isoindol-1-one,
- 3-amino-4,7-dichloro-isoindol-1-one,
- 3-amino-4,5,7-trichloro-isoindol-1-one,
- 3-amino-4,5,6,7-tétrachloro-isoindol-1-one,
- 3-amino-4,5,7-trichloro-6-méthylsulfanyl-isoindol-1-one,
- 3-amino-4,5,6,7-tétrabromo-isoindol-1-one,
- 3-amino-4,5,6,7-tétrafluoro-isoindol-1-one,
- 3-méthylamino-isoindol-1-one,
- 3-éthylamino-isoindol-1-one,
- 3-propylamino-isoindol-1-one,
- 3-diméthylamino-isoindol-1-one,
- 7-éthylamino-pyrrolo[3,4-b]pyridin-5-one,
- 7-amino-pyrrolo[3,4-b]pyridin-5-one,
- 3-amino-pyrrolo[3,4-c]pyridin-5-one,
- 3-amino-6-méthyl-pyrrolo[3,4-c]pyridin-1-one,
- 5-amino-pyrrolo[3,4-b]pyridin-7-one,
- 7-amino-pyrrolo[3,4-b]pyrazin-5-one,
- 7-amino-2-méthyl-pyrrolo[3,4-b]pyrazin-5-one,
- 7-amino-2,3-diméthyl-pyrrolo[3,4-b]pyrazin-5-one,
- 7-amino-2,3-dihydro-[1,4]dithiino[2,3-c]pyrrol-5-one,
- 3-imino-2-méthyl-2,3-dihydro-isoindol-1-one,
- 3-imino-2-éthyl-2,3-dihydro-isoindol-1-one,
- 3-imino-2-propyl-2,3-dihydro-isoindol-1-one,
- 2-hydroxyméthyl-3-imino-2,3-dihydro-isoindol-1-one,
- 2-(2-hydroxyéthyl)-3-imino-2,3-dihydro-isoindol-1-one,
- acide 2-(1-imino-3-oxo-1,3-dihydro-isoindol-2-yl)-éthane sulfonique,
- acide 3-(1-imino-3-oxo-1,3-dihydro-isoindol-2-yl)-propionique,
- 2-(3-hydroxypropyl)-3-imino-2,3-dihydro-isoindol-1-one,
- 5-imino-6-méthyl-5,6-dihydro-pyrrolo[3,4-b]pyridin-7-one,
et leurs sels cosmétiquement acceptables.

Parmi les composés à fonction amine primaire ou secondaire utilisables dans les compositions de teinture selon la présente invention, on peut notamment citer :
- des amines aromatiques telles que la N,N-bis-(2-hydroxyéthyl)-p-phénylènediamine, la 2-(2-hydroxyéthyl)-p-phénylènediamine, la N-(2-hydroxyéthyl)-N-éthyl-p-phénylènediamine, la N-(2-méthoxyéthyl)-p-phénylènediamine, la 2-chloro-p-phénylènediamine, les 2-, 3-, et 4-aminophénols, les o-, m-, et p- phénylènediamines, le 2,5-diaminotoluène, le 2,5-diaminophénol, le 2,5-diaminoanisol, la 4-méthylaminoaniline, la 3,4-diméthylaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-amino-6-chloro-phénol, le 2-méthyl-5-amino-4-chloro-phénol, le 1,3-diamino-2,4-diméthoxybenzène, les acides 2-, 3-, et 4-amino-benzoïques, les acides 2-, 3-, et 4-amino-phénylacétiques, les acides 2,3-, 2,4-, 2,5-, 3,4-, et 3,5-diamino-benzoïques, les acides 4-, et 5-amino-salicyliques, l'acide 3-amino-4-hydroxy-benzoïque, l'acide 4-amino-3-hydroxy-benzoïque, les acides 2-, 3-, et 4-amino-benzène sulfoniques, l'acide 3-amino-4-hydroxy-benzène sulfonique, l'acide 4-amino-3-hydroxy-naphtalène-1-sulfonique, l'acide 7-amino-4-hydroxy-naphtalène-2-sulfonique, l'acide 4-amino-5-hydroxy-naphtalène-2,7-disulfonique, l'acide 3-amino-2-naphtoïque, l'acide 3-amino-phtalique, l'acide 5-amino-isophtalique, les 1,3,5-, et 1,2,4-triaminobenzènes, le 1,2,4,5-tétraaminobenzène, le 2,4,5-triaminophénol, le pentaaminobenzène, l'hexaaminobenzène, la 2,4,6-triaminorésorcine, le 4,5-diamino-pyrocatéchol, le 4,6-diamino-pyrogallol, le 3,5-diamino-4-hydroxypyrocatéchol, le 2-méthyl-5-(2-hydroxyéthylamino)-phénol, le 2-méthoxy-3,5-diméthyl-4-aminobenzène, le 2,6-diméthyl-4-bis-(2-hydroxyéthyl)-amino-1-aminobenzène, le 5,6-diméthoxy-1,3-diaminobenzène, le 2,6-diméthyl-1,3-diaminobenzène, le 2,6-diméthoxy-5-chloro-1,3-diaminobenzène, le 2,6-diméthoxy-3-(2-hydroxyéthyl)-amino-1-aminobenzène, le 2,4-diméthoxy-3-(2-hydroxyéthyl)-amino-1-aminobenzène, le 4,6-dibenzyloxy-1,3-diaminobenzène, le 3-méthyl-6-méthoxy-1,2-diaminobenzène, le 3,5-diméthyl-4-aminophénol, le 2,5-diméthyl-4-aminophénol, le 2,3,5-triméthyl-4-aminophénol, le 2,3,5,6-tétraméthyl-4-aminophénol, le 4-chloro-5-acétylamino-2-aminophénol, le 4,6-diphényloxy-1,3-diaminobenzène, le 2,6-diméthyl-1,4-diaminobenzène, le 2,5-diméthyl-4-aminobenzène, le 2,3-diméthyl-4-aminobenzène, le 2-méthoxy-5-méthyl-1,4-diaminobenzène, le 2-méthoxy-5-méthyl-4-(2-aminoéthyl)-amino-1-aminobenzène, le 2-méthyl-5-chloro-4-(2-aminoéthyl)-amino-1-aminobenzène, le 4,6-diméthoxy-1,3-diaminobenzène, le 2,3-diméthyl-4-aminophénol, le 2,6-diméthyl-4-aminophénol, le 2,5-diméthyl-3-aminophénol, le **N**,**N'**-bis-(2-hydroxyéthyl)-**N**,**N'**-bis-(4-aminophényl)-1,3-diamino-2-propanol, la **N,N'**-bis-(2-hydroxyéthyl)-**N**,**N'**-bis-(4-aminophényl)-éthylènediamine, la **N**,**N'**-bis-(4-aminophényl)-tétraméthylènediamine, la **N**,**N'**-bis-(2-hydroxyéthyl)-**N**,**N'**-bis-(4-aminophényl)-tétraméthylènediamine, la **N**,**N'**-bis-(éthyl)-**N**,**N'**-bis-(4-amino-3-méthylphényl)-éthylènediamine, et leurs sels cosmétiquement acceptables, et,
- des amines hétérocycliques telles que, les 2-, 3-, et 4-amino-pyridines, la 2-amino-3-hydroxy-pyridine, les 2,3-, 2,5-, 2,6- et 3,4-diamino-pyridines, la 2-diméthylamino-5-amino-pyridine, la 3-amino-2-méthylamino-6-méthoxy-pyridine, la 2,3-diamino-6-méthoxy-pyridine, les 2,4,5-, et 4,5,6-triamino-pyrimidines, la 2,6-dihydroxy-3,4-diméthyl-pyridine, la 2,6-diméthoxy-3,5-diamino-pyridine, la 6-méthoxy-2,3-diamino-pyridine, la4-hydroxy-2,5,6-triamino-pyrimidine, la 2,4,5,6-tétraamino-pyrimidine, la 2-méthylamino-4,5,6-triamino-pyrimidine, les 2,4- et 4,5-diamino-pyrimidines, la 2-amino-4-méthoxy-6-méthyl-pyrimidine, la 5,6-diamino-2,4-dihydroxy-pyrimidine, le 3,4-diamino-thiophène, le 3,5-diamino-pyrazole, le 1-méthyl-4,5-diamino-pyrazole, le 1-éthyl-4,5-diamino-pyrazole, le 1,3-diméthyl-4,5-diamino-pyrazole, le 1-éthyl-3-méthyl-4,5-diamino-pyrazole, le 1-(4'-chloro-benzyl)-4,5-diamino-pyrazole, la 3-amino-pyrazoline, le 1H-1,2,4-triazole, le 3-amino-pyrazole, le 3-amino-5-hydroxy-pyrazole, les 2-, 3-, et 8-amino-quinoléines, la 5-amino-isoquinoléine, les acides 2-, et 6-amino-nicotiniques, les 4-, 5-, 6-, et 7-amino-indoles, le 2,3-diméthyl-5-amino-6-hydroxy-indole, le 2,3-diméthyl-5-amino-6-méthoxy-indole, le 2,3-diméthyl-5-chloro-6-amino-indole, le 2,3,4,5-tétraméthyl-6-amino-indole, le 2,3-diméthyl-5-méthoxy-6-amino-indole, le 2,3-diméthyl-5-éthyl-6-amino-indole, le 2-méthyl-6-amino-indole, le 2,3-diméthyl-5-hydroxy-6-amino-indole, le 2,3,5-triméthyl-6-amino-indole, le 2-méthyl-5-hydroxy-6-amino-indole, le 2,3-diméthyl-6-amino-indole, le 2,3,7-triméthyl-6-amino-indole, le 2,3,4-triméthyl-6-amino-indole, les 5-, et 6-amino-indazoles, les 5-, et 7-amino-benzimidazoles, les 5-, et 7-amino-benzothiazoles, la 2,5-dihydroxy-4-morpholinoaniline, le 5,6-dihydroxyindole, la 5,6-dihydroxyindoline, la 4-hydroxyindoline, la 6-aminoindoline, la N-éthyl-6-aminoindoline, les 3-, 4-, 5-, 6-, et 7-amino-indazoles , les 1-méthyl 3-, 4-, 5-, 6-, et 7-amino-indazoles, les 2-méthyl 3-, 4-, 5-, 6-, et 7-amino-indazoles, la pyrazolo-[1,5-a]-pyrimidine-6,7-diamine, le 5,6-diméthyl-pyrazolo-[1,5-a]-pyrimidine-3,7-diamine, le 2,6-diméthyl-pyrazolo-[1,5-a]-pyrimidine-3,7-diamine, le 2, 5, **N** 7, **N** 7-tétraméthyl-pyrazolo-[1,5-a]-pyrimidine-3,7-diamine, le 2,3-diméthyl-pyrazolo-[1,5-a]-pyrimidine-6,7-diamine, le 6-amino-5-méthyl-pyrazolo-[1,5-a]-pyrimidin-7-ol, le pyrazolo-[1,5-a]-pyrimidine-3,7-diamine, le 2,5-diméthyl-pyrazolo-[1,5-a]-pyrimidine-3,7-diamine, le pyrazolo-[1,5-a]-pyrimidine-3,5-diamine, le 2,7-diméthyl-pyrazolo-[1,5-a]-pyrimidine-3,5-diamine, le 3-amino-pyrazolo-[1,5-a]-pyrimidin-7-ol, le 3-amino pyrazolo-[1,5-a]-pyrimidin-5-ol,- le 2-(3-amino-pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo-[1,5-a]-pyrimidin-7-yl)-(2-hydroxy-éthyl)-amino]-éthanol, le 2-[(7-amino-pyrazolo-[1,5-a]-pyrimidin-3-yl)-(2-hydroxy-éthyl)-amino]-éthanol, et leurs sels cosmétiquement acceptables, et,
- des aminoacides, ou des oligopeptides comportant de 2 à 9 aminoacides, d'origine naturelle ou synthétique et obtenus par hydrolyse de protéines de plantes ou de protéines animales, comme par exemple le collagène, la kératine, la caséine, l'élastine, la protéine de soja, le gluten de blé, ou la protéine d'amande. Les acides aminés préférés sont la tyrosine, l'histidine, la lysine, la phénylalanine, l'ornithine, la dopa, l'arginine, et le tryptophane.

Au sens de la présente invention, les sels cosmétiquement acceptables des composés de formule (I) et des amines précitées peuvent être des chlorhydrates, des sulfates, des bromhydrates ou des tartrates.

La concentration en composé de formule (I) est de préférence comprise entre environ 0,01 et 5%, et encore plus préférentiellement entre environ 0,15 et 2 % en poids, par rapport au poids total de la composition tinctoriale.

La concentration en composé à fonction amine primaire ou secondaire est de préférence comprise entre environ 0,01 et 5 %, et encore plus préférentiellement entre environ 0,15 et 2 % en poids, par rapport au poids total de la composition tinctoriale.

Le milieu appruprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, par exemple, l'éthylèneglycol et ses éthers monométhylique, monoéthylique et monobutylique, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 20%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 2 à 11 et de préférence de 5 à 10, et pouvant être ajusté au moyen d'agents d'alcalinisation ou d'agents d'acidification ou de tampons antérieurement bien connus. Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 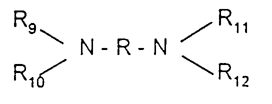 dans laquelle, R est un reste propylène éventuellement substitué par un groupement hydroxyle ou un radical alkyle en C₁-C₄ ; R₉ R₁₀, R₁₁ et R₁₂, simultanément ou indépendamment l'un de l'autre, représentent un atome d'hydrogène, un radical alkyle en C₁-C₄ ou hydroxyalkyle en C₁-C₄.
Les agents acidifiants sont classiquement des acides minéraux ou organiques comme par exemple les acides chlorhydrique, tartrique, citrique et phosphorique.
Parmi les tampons, on peut citer par exemple, le phosphate diacide de potassium/hydroxyde de sodium.

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Un autre objet de la présente invention porte sur un procédé de teinture des matières kératiniques, en particulier des fibres kératiniques humaines telles que les cheveux, consistant à appliquer une composition tinctoriale renfermant, dans un milieu approprié pour la teinture, au moins un dérivé de la di-imino-isoindoline ou de la 3-amino-isoindolone ou sa forme tautomère de formule (I) et un composé à fonction amine primaire ou secondaire, sur les fibres kératiniques sèches ou humides, à laisser agir la composition sur les fibres pendant un temps de pose variant entre 3 et 60 minutes environ, de préférence entre 5 et 45 minutes environ, à rincer, éventuellement laver, puis à rincer à nouveau, et à sécher.

Une variante de procédé constitue un autre objet de l'invention, et consiste à appliquer sur les fibres kératiniques, simultanément ou séquentiellement (i) une composition tinctoriale renfermant, dans un milieu approprié pour la teinture, au moins un dérivé de la di-imino-isoindoline ou de la 3-amino-isoindolone ou sa forme tautomère de formule (I) et (ii) une composition renfermant essentiellement un composé à fonction amine primaire ou secondaire dans un milieu approprié pour la teinture.

Des exemples concrets illustrant l'invention vont maintenant être donnés.

### EXEMPLE 1 :

On a préparé juste avant emploi la composition de teinture suivante :

| | |
|---|---|
| 3-imino-3H-isoindol-1-ylamine | 0,435 g |
| 2-(2-hydroxyéthyl)-p-phénylènediamine, HCl | 0,675 g |
| alcool benzylique | 10,0 g |
| alcool cétyl stéarylique 50/50 | 8,9 g |
| cétyl stéaryl sulfate de sodium | 8,9 g |
| eau q.s.p | 100, g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance orange.

### EXEMPLE 2 :

On a préparé juste avant emploi la composition de teinture suivante :

| | |
|---|---|
| 3-amino-isoindol-1-one | 0,438 g |
| 2-(2-hydroxyéthyl)-p-phénylènediamine, HCl | 0,675 g |
| alcool benzylique | 10,0 g |
| alcool cétyl stéarylique 50/50 | 8,9 g |
| cétyl stéaryl sulfate de sodium | 8,9 g |
| eau q.s.p | 100, g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance jaune.

### EXEMPLE 3 :

On a préparé juste avant emploi la composition de teinture suivante :

| | |
|---|---|
| 3-imino-3H-isoindol-1-ylamine | 0,435 g |
| sulfate de **N**,**N**-bis-(2-hydroxyéthyl)-p-phénylène-diamine | 0,588 g |
| alcool benzylique | 10,0 g |
| alcool cétyl stéarylique 50/50 | 8,9 g |
| cétyl stéaryl sulfate de sodium | 8,9 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance bois de rose.

### EXEMPLE 4 :

On a préparé juste avant emploi la composition de teinture suivante :

| | |
|---|---|
| 3-amino-isoindol-1-one | 0,438 g |
| sulfate de **N**,**N**-bis-(2-hydroxyéthyl)-p-phénylène-diamine | 0,588 g |
| alcool benzylique | 10,0 g |
| alcool cétyl stéarylique 50/50 | 8,9 g |
| cétyl stéaryl sulfate de sodium | 8,9 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune paille grisâtre.

### EXEMPLE 5 :

On a préparé juste avant emploi la composition de teinture suivante :

| | |
|---|---|
| 3-imino-3H-isoindol-1-ylamine | 0,435 g |
| 2,6-diamino-pyridine, 2HCl | 0,546 g |
| alcool ethylique | 30,0 g |
| eau q.s.p | 100, g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance jaune paille.

### EXEMPLE 6 :

On a préparé juste avant emploi la composition de teinture suivante :

| | |
|---|---|
| 3-imino-3H-isoindol-1-ylamine | 0,435 g |
| 3,7-diamino -pyrazolo[1,5-a]pyrimidine, 2HCl | 0,666 g |
| alcool ethylique | 30,0 g |
| eau q.s.p | 100, g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance jaune orangé.

### EXEMPLE 7 :

On a préparé juste avant emploi la composition de teinture suivante :

| | |
|---|---|
| 3-imino-3H-isoindol-1-ylamine | 0,435 g |
| 3-aminopyrazoline, 2HCl | 0,747 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance vert grisâtre.

### EXEMPLE 8 :

On a préparé juste avant emploi la composition de teinture suivante :

| | |
|---|---|
| 3-imino-3H-isoindol-1-ylamine | 0,435 g |
| 1-(4'-chlorobenzyl)-4,5-diamino-pyrazole, 2HCl | 0,886 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance orangé.

### EXEMPLE 9 :

On a préparé juste avant emploi la composition de teinture suivante :

| | |
|---|---|
| 3-imino-3H-isoindol-1-ylamine | 0,435 g |
| 5-aminoindole | 0,396 g |
| alcool éthylique | 30,0 g |
| eau q.s.p | 100, g |

On a appliqué la composition ci-dessus sur des mèches de cheveux gris à 90% de blancs et on a laissé poser pendant 30 minutes. Après rinçage à l'eau courante et séchage, les cheveux ont été teints dans une nuance orangé jaune.

### EXEMPLE 10:

On a préparé juste avant emploi la composition de teinture suivante :

| | |
|---|---|
| 3-imino-3H-isoindol-1-ylamine | 0,435 g |
| 2-méthyl-7-amino-indazole, HCl | 0,551 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune léger.

### EXEMPLE 11 :

On a préparé juste avant emploi la composition de teinture suivante :

| | |
|---|---|
| 3-imino-3H-isoindol-1-ylamine | 0,435 g |
| 3,4-diamino-thiophène, 2HBr | 0,828 g |
| alcool ethylique | 30,0 g |
| eau q.s.p | 100, g |

On a appliqué la composition ci-dessus sur des mèches de cheveux gris à 90% de blancs et on a laissé poser pendant 30 minutes. Après rinçage à l'eau courante et séchage, les cheveux ont été teints dans une nuance vert bronze.

### EXEMPLE 12:

On a préparé juste avant emploi la composition de teinture suivante :

| | |
|---|---|
| 3-imino-3H-isoindol-1-ylamine | 0,435 g |
| 1,3-diméthyl-4,5-diamino-pyrazole, 2HCl | 0,597 g |
| alcool éthylique | 30,0 g |
| eau q.s.p | 100, g |

On a appliqué la composition ci-dessus sur des mèches de cheveux gris à 90% de blancs et on a laissé poser pendant 30 minutes. Après rinçage à l'eau courante et séchage, les cheveux ont été teints dans une nuance jaune orangé.

### EXEMPLE 13:

On a préparé juste avant emploi la composition de teinture suivante :

| | |
|---|---|
| 3-imino-3H-isoindol-1-ylamine | 0,435 g |
| 1-méthyl-4,5-diamino-pyrazole, 2HCl | 0,555 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance orangé flamboyant.

### EXEMPLE 14 :

On a préparé juste avant emploi la composition de teinture suivante :

| | |
|---|---|
| 3-imino-3H-isoindol-1-ylamine | 0,435 g |
| 3-méthyl-4-amino-phénol, HBr | 0,612 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune.

### EXEMPLE 15 :

On a préparé juste avant emploi la composition de teinture suivante :

| | |
|---|---|
| 3-imino-3H-isoindol-1-ylamine | 0,435 g |
| 4-aminoindazole, 2HCl | 0,618 g |
| alcool éthylique | 30,0 g |
| eau q.s.p | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance beige rosé.

## Revendications

1. Utilisation d'un composé de formule (I): 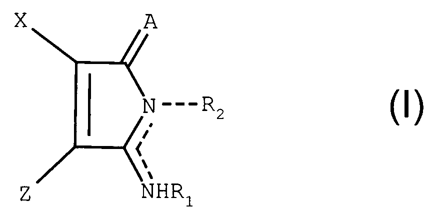 dans laquelle,
**R**_{**1**} et **R**_{**2**} désignent, indépendamment l'un de l'autre, H, alkyle, hydroxyalkyle, polyhydroxyalkyle, alkylhydroxyalkyle, aminoalkyle (l'amine pouvant être protégée par un radical acétyle, uréido, sulfonyle), alkylaminoalkyle, (dihydroxy)alkylaminoalkyle, ou alkyle-NR'R'' (dans lequel R' et R'' sont alkyle ou peuvent former ensemble avec l'atome d'azote auxquels ils sont rattachés, un cycle aliphatique ou hétérocyclique à 5 ou 6 chaînons),
étant entendu que tous les radicaux alkyle des groupements ci-avant définis comportent de 1 à 4 atomes de carbone et peuvent être linéaires ou ramifiés, et que R₂ peut être nul,
**A** désigne O ou NH,
**X** et **Z** forment ensemble un cycle hydrocarboné à 5 ou 6 chaînons, saturé ou insaturé, aromatique ou hétérocyclique pouvant être interrompu par un ou plusieurs atomes d'azote ou de soufre, et qui peut être substitué par un ou plusieurs radicaux tels que NO₂, NH₂, acétylamino, OH, SO₃H, halogène (Br, Cl, F), CH₃SO₂, -CF₃, -OCF₃, alkyle en C₁-C₄, alcoxy(C₁-C₄), alkyl(C₁-C₄)thio, alcoxy (C₁-C₄)carbonyle,
et les sels cosmétiquement acceptables de ces composés,
comme précurseur de coloration susceptible de produire une coloration par réaction, sans agent oxydant, avec un composé à fonction amine primaire ou secondaire, dans une, ou pour la préparation d'une, composition de teinture des fibres kératiniques.

2. Utilisation selon la revendication 1, selon laquelle le composé de formule (I) est choisi dans le groupe constitué par :
- 3-imino-3H-isoindol-ylamine,
- 3-imino-4-méthyl-3H-isoindol-1-lamine,
- 3-imino-4-terbutyl-3H-isoindol-1-ylamine,
- 3-imino-7-nitro-3H-isoindol-1-ylamine,
- 3-amino-1-imino-1H-isoindol-4-ol,
- 3-imino-7-isopropoxy-3H-isoindol-1-ylamine,
- 3-imino-7-(2,2,2-trifluoroéthoxy)-3H-isoindol-1-ylamine,
- 3-imino-7-éthoxy-3H-isoindol-1-ylamine,
- 3-imino-7-butoxy-3H-isoindol-1-ylamine,
- acide 3-amine-1-imino-1 H-isoindole-4-sulfonique,
- 3-imino-7-chloro-3H-isoindol-1-ylamine,
- 3-imino-5-méthyl-3H-isoindol-1-ylamine,
- 3-imino-5-éthyl-3H-isoindol-1-ylamine,
- 3-imino-5-terbutyl-3H-isoindol-1-ylamine,
- 3-imino-5-amino-3H-isoindol-1-ylamine,
- N-(1-amino-3-imino-3H-isoindol-5-yl)-acétamide,
- 3-imino-5-nitro-3H-isoindol-1-ylamine,
- 3-imino-5-fluoro-3H-isoindol-1-ylamine,
- 3-imino-5-chloro-3H-isoindol-1-ylamine,
- 3-imino-5-méthylsulfanyl-3H-isoindol-1-ylamine,
- 3-imino-5-méthoxy-3H-isoindol-1-ylamine,
- 3-imino-5-éthoxy-3H-isoindol-1-ylamine,
- 3-imino-5-propoxy-3H-isoindol-1-ylamine,
- 3-imino-5-isopropoxy-3H-isoindol-1-ylamine,
- 3-imino-5-butoxy-3H-isoindol-1-ylamine,
- 3-imino-5-isobutoxy-3H-isoindol-1-ylamine,
- 3-imino-5-terbutoxy-3H-isoindol-1-ylamine,
- 3-imino-5-(2,2,2-trifluorométhyl)-3H-isoindol-1-ylamine,
- 3-imino-5-(2,2,2-trifluoroéthoxy)-3H-isoindol-1-ylamine,
- 3-imino-5-méthanesulfonyl-3H-isoindol-1-ylamine,
- 3-imino-5,6-diméthyl-3H-isoindol-1-ylamine,
- 3-imino-5,6-diéthyl-3H-isoindol-1-ylamine,
- 3-imino-5,6-diméthoxy-3H-isoindol-1-ylamine,
- 3-imino-5,6-diéthoxy-3H-isoindol-1-ylamine,
- 3-imino-5,6-dibutoxy-3H-isoindol-1-ylamine,
- 3-imino-5,6-bis-trifluorométhyl-3H-isoindol-1-ylamine,
- 3-imino-5,6-dichloro-3H-isoindol-1-ylamine,
- 5,6-bis-éthoxyméthyl-3-imino-3H-isoindol-1-ylamine,
- 3-amino-1-imino-1H-isoindole-4,7-diol,
- 4,7-dichloro-3-imino-3H-isoindol-1-ylamine,
- 4,5,7-trichloro-3-imino-N6,N6-diméthyl-3H-isoindole-1,6-diamine,
- 4,5,6,7-tétrachloro-3-imino-3H-isoindol-1-ylamine,
- 4,5,6,7-tétrafluoro-3-imino-3H-isoindol-1-ylamine,
- 3-butylimino-3H-isoindol-1-ylamine,
- 2-(3-amino-isoindol-1-ylidèneamino)-éthanol,
- 3-(3-amino-isoindol-1-ylidèneamino)-3-méthyl-pentane-1,5-diol,
- N-(3-amino- isoindol-1-ylidène)-guanidine,
- 7-imino-7H-pyrrolo[3,4-b]pyridin-5-ylamine,
- 7-imino-7H-pyrrolo[3,4-b]pyrazin-5-ylamine,
- 7-imino-2,3-diméthyl-7H-pyrrolo[3,4-b]pyrazin-5-ylamine,
- 7-imino-7H-[1,4]dithiino[2,3-c]pyrrol-5-ylamine,
- 7-imino-2,3-diméthyl-7H-[1,4]dithiino[2,3-c]pyrrol-5-ylamine,
- 7-imino-2,3-dihydro-7H-[1,4]dithiino[2,3-c]pyrrol-5-ylamine,
- 7-imino-2-méthyl-2,3-dihydro-7H-[1,4]dithiino[2,3-c]pyrrol-5-ylamine,
- 3-amino-isoindol-1-one,
- 3-amino-7-méthyl-isoindol-1-one,
- 3-amino-7-hydroxyméthyl-isoindol-1-one,
- 3-amino-7-chloro-isoindol-1-one,
- 3-amino-4-chloro-isoindol-1-one,
- acide 3-amino-1-oxo-1H-isoindole-4-sulfonique,
- 3-amino-4-nitro-isoindol-1-one,
- 3-amino-6-nitro-isoindol-1-one,
- 3-amino-6-méthyl-isoindol-1-one,
- 3-amino-6-chloro-isoindol-1-one,
- 3-amino-6-bromo-isoindol-1-one,
- 3-amino-6-méthylsulfanyl-isoindol-1-one,
- 3-amino-6-méthoxy-isoindol-1-one,
- 3-amino-5-chloro-isoindol-1-one,
- 3-amino-5-fluoro-isoindol-1-one,
- 3-amino-5-méthoxy-isoindol-1-one,
- 3-amino-5-nitro-isoindol-1-one,
- ester éthylique de l'acide 3-amino-1-oxo-1H-isoindole-5-carboxylique,
- 3-amino-5,6-dichloro-isoindol-1-one,
- 3-amino-5,6-dibromo-isoindol-1-one,
- 3-amino-4,7-dichloro-isoindol-1-one,
- 3-amino-4,5,7-trichloro-isoindol-1-one,
- 3-amino-4,5,6,7-tétrachloro-isoindol-1-one,
- 3-amino-4,5,7-trichloro-6-méthylsulfanyl-isoindol-1-one,
- 3-amino-4,5,6,7-tétrabromo-isoindol-1-one,
- 3-amino-4,5,6,7-tétrafluoro-isoindol-1-one,
- 3-méthylamino-isoindol-1-one,
- 3-éthylamino-isoindol-1-one,
- 3-propylamino-isoindol-1-one,
- 3-diméthylamino-isoindol-1-one,
- 7-éthylamino-pyrrolo[3,4-b]pyridin-5-one,
- 7-amino-pyrrolo[3,4-b]pyridin-5-one,
- 3-amino-pyrrolo[3,4-c]pyridin-5-one,
- 3-amino-6-méthyl-pyrrolo[3,4-c]pyridin-1-one,
- 5-amino-pyrrolo[3,4-b]pyridin-7-one,
- 7-amino-pyrrolo[3,4-b]pyrazin-5-one,
- 7-amino-2-méthyl-pyrrolo[3,4-b]pyrazin-5-one,
- 7-amino-2,3-diméthyl-pyrrolo[3,4-b]pyrazin-5-one,
- 7-amino-2,3-dihydro-[1,4]dithiino[2,3-c]pyrrol-5-one,
- 3-imino-2-méthyl-2,3-dihydro-isoindol-1-one,
- 3-imino-2-éthyl-2,3-dihydro-isoindol-1-one,
- 3-imino-2-propyl-2,3-dihydro-isoindol-1-one,
- 2-hydroxyméthyl-3-imino-2,3-dihydro-isoindol-1-one,
- 2-(2-hydroxyéthyl)-3-imino-2,3-dihydro-isoindol-1-one,
- acide 2-(1-imino-3-oxo-1,3-dihydro-isoindol-2-yl)-éthane sulfonique,
- acide 3-(1-imino-3-oxo-1,3-dihydro-isoindol-2-yl)-propionique,
- 2-(3-hydroxypropyl)-3-imino-2,3-dihydro-isoindol-1-one,
- 5-imino-6-méthyl-5,6-dihydro-pyrrolo[3,4-b]pyridin-7-one,
et leurs sels cosmétiquement acceptables.

3. Utilisation selon la revendication 1 ou 2, caractérisée par le fait que les fibres kératiniques sont humaines.

4. Utilisation selon la revendication 3, caractérisée par le fait qu'il s'agit des cheveux.

5. Composition de teinture des fibres kératiniques, en particulier des fibres kératiniques humaines telles que les cheveux, caractérisée par le fait qu'elle comprend, dans un milieu approprié pour la teinture, (i) au moins un composé de formule (I) défini selon les revendications 1 ou 2, et, (ii) un composé à fonction amine primaire ou secondaire.

6. Composition de teinture selon la revendication 5, caractérisée par le fait que le composé à fonction amine primaire ou secondaire est de type aromatique ou hétérocyclique.

7. Composition de teinture selon la revendication 5, caractérisée par le fait que le composé à fonction amine primaire ou secondaire est un acide aminé ou un oligopeptide comportant de 2 à 9 aminoacides.

8. Composition de teinture selon l'une quelconque des revendications 5 à 7, caractérisée par le fait qu'elle a un pH compris entre 2 et 11.

9. Composition selon l'une quelconque des revendications 5 à 8, caractérisée par le fait que le composé de formule (I) est présent dans une concentration allant de 0,01 à 5% en poids par rapport au poids total de la composition.

10. Composition selon l'une quelconque des revendications 5 à 9, caractérisée par le fait que le composé à fonction amine primaire ou secondaire est présent dans une concentration allant de 0,01 à 5% en poids par rapport au poids total de la composition.

11. Composition selon l'une quelconque des revendications 5 à 10, caractérisée par le fait que le milieu approprié pour la teinture est un milieu aqueux constitué par de l'eau et/ou des solvants organiques choisis parmi les alcools, les glycols et les éthers de glycol, dans des proportions comprises entre 0,5 et 20% en poids par rapport au poids total de la composition.

12. Composition de teinture à deux composants pour lesquels, dans un milieu approprié pour la teinture, l'un contient au moins un composé de formule (I) défini aux revendications 1 et 2, l'autre un composé à fonction amine primaire ou secondaire, et qui, stockés de façon séparée, sont (i) mélangés au moment de l'emploi pour l'application sur les fibres kératiniques ou (ii) appliqués séquentiellement sur lesdites fibres.

13. Procédé de teinture des fibres kératiniques et en particulier des fibres kératiniques humaines telles que les cheveux, caractérisé par le fait qu'on applique la composition tinctoriale définie selon l'une quelconque des revendications 5 à 12, sur les fibres kératiniques sèches ou humides, et qu'après avoir laissé agir la composition pendant 3 à 60 minutes environ, on rince les fibres, on les lave éventuellement, on les rince à nouveau puis on les sèche.

14. Dispositif à plusieurs compartiments, ou «kits», pour la teinture des fibres kératiniques, caractérisé par le fait qu'il comporte au moins deux compartiments, dont l'un d'eux renferme une composition contenant, dans un milieu approprié pour la teinture, au moins un composé de formule (I) tel que défini dans les revendications 1 et 2, et l'autre renferme une composition contenant, dans un milieu approprié pour la teinture, un composé à fonction amine primaire ou secondaire tel que défini dans l'une quelconque des revendications 1, 5 à 7.

## Claims

1. Use of a compound of formula (I): 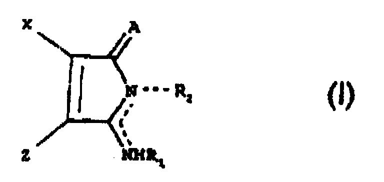 in which,
R₁ and R₂ denote, independently of each other, H, alkyl, hydroxyalkyl, polyhydroxyalkyl, alkylhydroxyalkyl, aminoalkyl (it being possible for the amine to be protested with an acetyl, ureido or sulphonyl radical), alkylaminoalkyl, (dihydroxy)alkylaminoalkyl or alkyl-NR'R'' (in which R' and R'' are alkyl or can form, together with the nitrogen atom to which they are attached, a 5- or 6-membered aliphatic or heterocyclic ring),
it being understood that all the alkyl radicals of the groups defined above contain from 1 to 4 carbon atoms and may be linear or branched, and that R₂ can be nothing,
A denotes O or NH,
X and Z together form a saturated or unsaturated, aromatic or heterocyclic, 5- or 6-membered hydrocarbon ring which may be interrupted by one or more nitrogen or sulphur atoms, and which can be substituted with one or more radicals such as NO₂, NH₂, acetylamino, OH, SO₃H, halogen (Br, Cl, F), CH₃SO₂, -CF₃, -OCF₃, C₁-C₄alkyl, (C₁-C₄)alkoxy, (C₁-C₄)alkylthio, (C₁-C₄)alkoxy-carbonyl,
and the cosmetically acceptable salts of these compounds,
as a dye precursor capable of producing a dyeing by reacting, without oxidizing agent, with a compound containing a primary or secondary amine function, in, or for the preparation of, a composition for dyeing keratin fibres.

2. Use according to Claim 1, according to which the compound of formula (I) is chosen from the group consisting of:
- 3-imino-3H-isoindolylamine,
- 3-imino-4-methyl-3H-isoindol-1-ylamine,
- 3-imino-4-tertbutyl-3H-isoindol-1-ylamine,
- 3-imino-7-nitro-3H-isoindol-1-ylamine,
- 3-amino-1-imino-1H-isoindol-4-ol,
- 3-imino-7-isopropoxy-3H-isoindol-1-ylamine,
- 3-imino-7-(2,2,2-trifluoroethoxy)-3H-isoindol-1-ylamine,
- 3-imino-7-ethoxy-3H-isoindol-1-ylamine,
- 3-imino-7-butoxy-3H-isoindol-1-ylamine,
- 3-amino-1-imino-1H-isoindole-4-sulphonic acid
- 3-imino-7-chloro-3H-isoindol-1-ylamine,
- 3-imino-5-methyl-3H-isoindol-1-ylamine,
- 3-imino-5-ethyl-3H-isoindol-1-ylamine,
- 3-imino-5-tertbutyl-3H-isoindol-1-ylamine,
- 3-imino-5-amino-3H-isoindol-1-ylamine,
- N-(1-amino-3-imino-3H-isoindol-5-yl)acetamide,
- 3-imino-5-nitro-3H-isoindol-1-ylamine,
- 3-imino-5-fluoro-3H-isoindol-1-ylamine,
- 3-imino-5-chloro-3H-isoindol-1-ylamine,
- 3-imino-5-methylsulphanyl-3H-isoindol-1-ylamine,
- 3-imino-5-methoxy-3H-isoindol-1-ylamine,
- 3-imino-5-ethoxy-3H-isoindol-1-ylamine,
- 3-imino-5-propoxy-3H-isoindol-1-ylamine,
- 3-imino-5-isopropoxy-3H-isoindol-1-ylamine,
- 3-imino-5-butoxy-3H-isoindol-1-ylamine,
- 3-imino-5-isobutoxy-3H-isoindol-1-ylamine,
- 3-imino-5-tertbutoxy-3H-isoindol-1-ylamine,
- 3-imino-5-(2,2,2-trifluoromethyl)-3H-isoindol-1-ylamine,
- 3-imino-5-(2,2,2-trifluoroethoxy)-3H-isoindol-1-ylamine,
- 3-imino-5-methanesulphonyl-3H-isoindol-1-ylamine,
- 3-imino-5,6-dimethyl-3H-isoindol-1-ylamine,
- 3-imino-5,6-diethyl-3H-isoindol-1-ylamine,
- 3-imino-5,6-dimethoxy-3H-isoindol-1-ylamine,
- 3-imino-5,6-diethoxy-3H-isoindol-1-ylamine,
- 3-imino-5,6-dibutoxy-3H-isoindol-1-ylamine,
- 3-imino-5,6-bis(trifluoromethyl)-3H-isoindol-1-ylamine,
- 3-imino-5,6-dichloro-3H-isoindol-1-ylamine,
- 5,6-bis(ethoxymethyl)-3-imino-3H-isoindol-1-ylamine,
- 3-amino-1-imino-1H-isoindole-4,7-diol,
- 4,7-dichloro-3-imino-3H-isoindol-1-ylamine,
- 4, 5, 7-trichloro-3-imino-N6,N6-dimethyl-3H-isoindole-1,6-diamine,
- 4,5,6,7-tetrachloro-3-imino-3H-isoindol-1-ylamine,
- 4,5,6,7-tetrafluoro-3-imino-3H-isoindol-1-ylamine,
- 3-butylimino-3H-isoindol-1-ylamine,
- 2-(3-aminoisoindol-1-ylideneamino)ethanol,
- 3-(3-aminoisoindol-1-ylideneamino)-3-methylpentane-1,5-diol,
- N-(3-aminoisoindol-1-ylidene)guanidine,
- 7-imino-7H-pyrrolo[3,4-b]pyrid-5-ylamine,
- 7-imino-7H-pyrrolo[3,4-b]pyrazin-5-ylamine,
- 7-imino-2,3-dimethyl-7H-pyrrolo[3,4-b]pyrazin-5-ylamine,
- 7-imino-7H[1,4]dithiino[2,3-c]pyrrol-5-ylamine,
- 7-imino-2,3-dimethyl-7H-[1,4]dithiino[2,3-c]pyrrol-5-ylamine,
- 7-imino-2,3-dihydro-7H-[1,4]dithiino[2,3-c]-pyrrol-5-ylamine,
- 7-imino-2-methyl-2,3-dihydro-7H-[1,4]dithiino-[2,3-c]pyrrol-5-ylamine,
- 3-aminoisoindol-1-one,
- 3-amino-7-methylisoindol-1-one,
- 3-amino-7-hydroxymethylisoindol-1-one,
- 3-amino-7-chloroisoindol-1-one,
- 3-amino-4-chloroisoindol-1-one,
- 3-amino-1-oxo-1H-isoindole-4-sulphonic acid,
- 3-amino-4-nitroisoindol-1-one,
- 3-amino-6-nitroisoindol-1-one,
- 3-amino-6-methylisoindol-1-one,
- 3-amino-6-chloroisoindol-1-one,
- 3-amino-6-bromoisoindol-1-one,
- 3-amino-6-methylsulphanylisoindol-1-one,
- 3-amino-6-methoxyisoindol-1-one,
- 3-amino-5-chloroisoindol-1-one,
- 3-amino-5-fluoroisoindol-1-one,
- 3-amino-5-methoxyisoindol-1-one,
- 3-amino-5-nitroisoindol-1-one,
- ethyl 3-amino-1-oxo-1H-isoindole-5-carboxylate
- 3-amino-5,6-dichloroisoindol-1-one,
- 3-amino-5,6-dibromoisoindol-1-one,
- 3-amino-4,7-dichloroisoindol-1-one,
- 3-amino-4,5,7-trichloroisoindol-1-one,
- 3-amino-4,5,6,7-tetrachloroisoindol-1-one,
- 3-amino-4,5,7-trichloro-6-methyl sulphanylisoindol-1-one,
- 3-amino-4,5,6,7-tetrabromoisoindol-1-one,
- 3-amino-4,5,6,7-tetrafluoroisoindol-1-one,
- 3-methylaminoisoindol-1-one,
- 3-ethylaminoisoindol-1-one,
- 3-propylaminoisoindol-1-one,
- 3-dimethylaminoisoindol-1-one,
- 7-ethylaminopyrrolo[3,4-b]pyrid-5-one,
- 7-aminopyrrolo[3,4-b]pyrid-5-one,
- 3-aminopyrrolo[3,4-c]pyrid-5-one,
- 3-amino-6-methylpyrrolo[3,4-c]pyrid-1-one,
- 5-aminopyrrolo[3,4-b]pyrid-7-one,
- 7-aminopyrrolo[3,4-b]pyrazin-5-one,
- 7-amino-2-methylpyrrolo[3,4-b]pyrazin-5-one,
- 7-amino-2,3-dimethylpyrrolo[3,4-b]pyrazin-5-one.
- 7-amino-2,3-dihydro-[1,4]dithiino[2,3-c]pyrrol-5-one,
- 3-imino-2-methyl-2,3-dihydroisoindol-1-one,
- 3-imino-2-ethyl-2,3-dihydroisoindol-1-one,
- 3-imino-2-propyl-2,3-dihydroisoindol-1-one,
- 2-hydroxymethyl-3-imino-2,3-dihydroisoindol-1-one,
- 2-(2-hydroxyethyl)-3-imino-2,3-dihydroisoindol-1-one,
- 2-(1-imino-3-oxo-1,3-dihydroisoindol-2-yl)ethane-sulphonic acid
- 3-(1-imino-3-oxo-1,3-dihydroisoindol-2-yl)propionic acid
- 2-(3-hydroxypropyl)-3-imino-2,3-dihydroisoindol-1-one,
- 5-imino-6-methyl-5,6-dihydropyrrolo[3,4-b]pyrid-7-one,
and the cosmetically acceptable salts thereof.

3. Use according to Claim 1 or 2, characterized in that the keratin fibres are human.

4. Use according to Claim 3, characterized in that it is hair.

5. Composition for dyeing keratin fibres, in particular human keratin fibres such as the hair, characterized in that it comprises, in a medium which is suitable for dyeing, (i) at least one compound of formula (I) defined according to Claims 1 or 2, and (ii) a compound containing a primary or secondary amine function.

6. Dye composition according to Claim 5, characterized in that the compound containing a primary or secondary amine function is of aromatic or heterocyclic type.

7. Dye composition according to Claim 5, characterized in that the compound containing a primary or secondary amine function is an amino acid or an oligopeptide containing from 2 to 9 amino acids.

8. Dye composition according to any one of Claims 5 to 7, characterized in that it has a pH of between 2 and 11.

9. Composition according to any one of Claims 5 to 8, characterized in that the compound of formula (I) is present in a concentration ranging from 0.01 to 5% by weight relative to the total weight of the composition.

10. Composition according to any one of Claims 5 to 9, characterized in that the compound containing a primary or secondary amine function is present in a concentration ranging from 0.01 to 5% by weight relative to the total weight of the composition.

11. Composition according to any one of Claims 5 to 10, characterized in that the medium which is suitable for dyeing is an agueous medium consisting of water and/or organic solvents chosen from alcohols, glycols and glycol ethers, in proportions of between 0.5 and 20% by weight relative to the total weight of the composition.

12. Two-component dye composition for which, in a medium which is suitable for dyeing, one component contains at least one compound of formula (I) defined in Claims 1 and 2, and the other contains a compound containing a primary or secondary amine function, and which, stored separately, are (i) mixed together, at the time of use, for application to the keratin fibres, or (ii) applied sequentially to the said fibres.

13. Process for dyeing keratin fibres, and in particular human keratin fibres such as the hair, characterized in that the dye composition defined according to any one of Claims 5 to 12 is applied to the wet or dry keratin fibres and, after the composition has been left to act for 3 to 60 minutes approximately, the fibres are rinsed, optionally washed, rinsed again and then dried.

14. Multi-compartment device, or "kit", for dyeing keratin fibres, characterized in that it contains at least two compartments, one of which holds a composition containing, in a medium which is suitable for dyeing, at least one compound of formula (I) as defined in Claims 1 and 2, and the other holds a composition containing, in a medium which is suitable for dyeing, a compound containing a primary or secondary amine function as defined in any one of Claims 1, 5 to 7.

## Patentansprüche

1. Verwendung einer Verbindung der Formel (I) 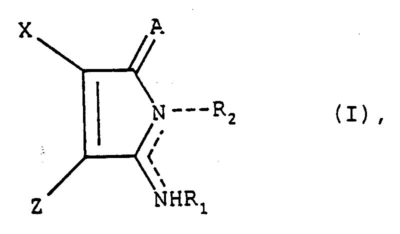 worin:
R₁ und R₂ unabhängig voneinander bedeuten: H, Alkyl, Hydroxyalkyl, Polyhydroxyalkyl, Alkylhydroxyalkyl, Aminoalkyl (wobei das Amin mit einer Acetylgruppe, Ureidogruppe oder Sulfonylgruppe geschützt sein kann), Alkylaminoalkyl, (Dihydroxy)alkylaminoalkyl oder Alkyl-NR'R'' (wobei es sich bei den Gruppen R' und R'' um Alkylgruppen handelt oder die Gruppen R' und R'' gemeinsam mit dem Stickstoffatom, an das sie gebunden sind, einen 5- oder 6-gliedrigen aliphatischen oder heterocyclischen Ring bilden können),
mit der Maßgabe, daß alle Alkylreste der oben definierten Gruppen 1 bis 4 Kohlenstoffatome aufweisen und geradkettig oder verzweigt vorliegen können, wobei R₂ auch nicht vorhanden sein kann,
A O oder NH bedeutet,
X und Z gemeinsam einen 5- oder 6-gliedrigen, gesättigten oder ungesättigten, aromatischen oder heterocyclischen Kohlenwasserstoffring bilden, der durch ein oder mehrere Stickstoffatome oder Schwefelatome unterbrochen sein kann und der mit einer oder mehreren Gruppen substituiert sein kann, wie beispielsweise NO₂, NH₂, Acetylamino, OH, SO₃H, Halogen (Br, Cl, F), CH₃SO₂, -CF₃, -OCF₃, C₁₋₄-Alkyl, C₁₋₄-Alkoxy, C₁₋₄-Alkylthio, C₁₋₄-Alkoxycarbonyl,
und der kosmetisch akzeptablen Salze dieser Verbindungen
als Farbstoffvorprodukte, die ohne Oxidationsmittel durch Reaktion mit einer Verbindung mit primärer oder sekundärer Aminogruppe eine Färbung hervorrufen können, in einer Zusammensetzung zum Färben von Keratinfasern oder zur Herstellung einer Zusammensetzung zum Färben von Keratinfasern.

2. Verwendung nach Anspruch 1, wobei die Verbindung der Formel (I) ausgewählt ist unter:
- 3-Imino-3H-isoindol-ylamin,
- 3-Imino-4-methyl-3H-isoindol-1-ylamin,
- 3-Imino-4-tert.-butyl-3H-isoindol-1-ylamin,
- 3-Imino-7-nitro-3H-isoindol-1-ylamin,
- 3-Amino-1-imino-1H-isoindol-4-ol,
- 3-Imino-7-isopropoxy-3H-isoindol-1-ylamin,
- 3-Imino-7-(2,2,2-trifluorethoxy)-3H-isoindol-1-ylamin,
- 3-Imino-7-ethoxy-3H-isoindol-1-ylamin,
- 3-Imino-7-butoxy-3H-isoindol-1-ylamin,
- 3-Amino-1-imino-1H-isoindol-4-sulfonsäure,
- 3-Imino-7-chlor-3H-isoindol-1-ylamin,
- 3-Imino-5-methyl-3H-isoindol-1-ylamin,
- 3-Imino-5-ethyl-3H-isoindol-1-ylamin,
- 3-Imino-5-tert.-butyl-3H-isoindol-1-ylamin,
- 3-Imino-5-amino-3H-isoindol-1-ylamin,
- N- (1-Amino-3-imino-3H-isoindol-5-yl)-acetamid,
- 3-Imino-5-nitro-3H-isoindol-1-ylamin,
- 3-Imino-5-fluor-3H-isoindol-1-ylamin,
- 3-Imino-5-chlor-3H-isoindol-1-ylamin,
- 3-Imino-5-methylsulfanyl-3H-isoindol-1-ylamin,
- 3-Imino-5-methoxy-3H-isoindol-1-ylamin,
- 3-Imino-5-ethoxy-3H-isoindol-1-ylamin,
- 3-Imino-5-propoxy-3H-isoindol-1-ylamin,
- 3-Imino-5-isopropoxy-3H-isoindol-1-ylamin,
- 3-Imino-5-butoxy-3H-isoindol-1-ylamin,
- 3-Imino-5-isobutoxy-3H-isoindol-1-ylamin,
- 3-Imino-5-tert.-butoxy-3H-isoindol-1-ylamin,
- 3-Imino-5-(2,2,2-trifluormethyl)-3H-isoindol-1-ylamin,
- 3-Imino-5-(2,2,2-trifluorethoxy)-3H-isoindol-1-ylamin,
- 3-Imino-5-methansulfonyl-3H-isoindol-1-ylamin,
- 3-Imino-5,6-dimethyl-3H-isoindol-1-ylamin,
- 3-Imino-5,6-diethyl-3H-isoindol-1-ylamin,
- 3-Imino-5,6-dimethoxy-3H-isoindol-1-ylamin,
- 3-Imino-5,6-diethoxy-3H-isoindol-1-ylamin,
- 3-Imino-5,6-dibutoxy-3H-isoindol-1-ylamin,
- 3-Imino-5,6-bis-trifluormethyl-3H-isoindol-1-ylamin,
- 3-Imino-5,6-dichlor-3H-isoindol-1-ylamin,
- 5,6-Bis-ethoxymethyl-3-imino-3H-isoindol-1-ylamin,
- 3-Amino-1-imino-1H-isoindol-4,7-diol,
- 4,7-Dichlor-3-imino-3H-isoindol-1-ylamin,
- 4,5,7-Trichlor-3-imino-N6,N6-dimethyl-3H-isoindol-1,6-diamin,
- 4,5,6,7-Tetrachlor-3-imino-3H-isoindol-1-ylamin,
- 4,5,6,7-Tetrafluor-3-imino-3H-isoindol-1-ylamin,
- 3-Butylimino-3H-isoindol-1-ylamin,
- 2-(3-Aminoisoindol-1-ylidenamino)-ethanol,
- 3-(3-Aminoisoindol-1-ylidenamino)-3-methyl-pentan-1,5-diol,
- N-(3-Aminoisoindol-1-yliden)-guanidin,
- 7-Imino-7H-pyrrolo[3,4-b]pyridin-5-ylamin,
- 7-Imino-7H-pyrrolo[3,4-b]pyrazin-5-ylamin,
- 7-Imino-2,3-dimethyl-7H-pyrrolo[3,4-b]pyrazin-5-ylamin,
- 7-Imino-7H-[1,4]dithiino[2,3-c]pyrrolo-5-ylamin,
- 7-Imino-2,3-dimethyl-7H-[1,4]dithiino[2,3-c]pyrrol-5-ylamin
- 7-Imino-2,3-dihydro-7H-[1,4]dithiino[2,3-c]pyrrol-5-ylamin,
- 7-Imino-2-methyl-2,3-dihydro-7H-[1,4]dithiino[2,3-c]-pyrrol-5-ylamin,
- 3-Amino-isoindol-1-on,
- 3-Amino-7-methyl-isoindol-1-on,
- 3-Amino-7-hydroxymethyl-isoindol-1-on,
- 3-Amino-7-chlor-isoindol-1-on,
- 3-Amino-4-chlor-isoindol-1-on,
- 3-Amino-1-oxo-1H-isoindol-4-sulfonsäure,
- 3-Amlno-4-nitro-isoindol-1-on,
- 3-Amino-6-nitro-isoindol-1-on,
- 3-Amino-6-methyl-isoindol-1-on,
- 3-Amino-6-chlor-isoindol-1-on,
- 3-Amino-6-brom-isoindol-1-on,
- 3-Amino-6-methylsulfanyl-isoindol-1-on,
- 3-Amino-6-methoxy-isoindol-1-on,
- 3-Amino-5-chlor-isoindol-1-on,
- 3-Amino-5-fluor-isoindol-1-on,
- 3-Amino-5-methoxy-isoindol-1-on,
- 3-Amino-5-nitro-isoindol-1-on,
- Ethylester von 3-Amino-1-oxo-1H-isoindol-5-carbonsäure,
- 3-Amino-5,6-dichlor-isoindol-1-on,
- 3-Amino-5,6-dibrom-isoindol-1-on,
- 3-Amino-4,7-dichlor-isoindol-1-on
- 3-Amino-4,5,7-trichlor-isoindol-1-on,
- 3-Amino-4,5,6,7-tetrachlor-isoindol-1-on,
- 3-Amino-4,5,7-trichlor-6-methylsulfanyl-isoindol-1-on,
- 3-Amino-4,5,6,7-tetrabrom-isoindol-1-on,
- 3-Amino-4,5,6,7-tetrafluor-isoindol-1-on,
- 3-Methylamino-isoindol-1-on,
- 3-Ethylamino-isoindol-1-on,
- 3-Propylamino-isoindol-1-on,
- 3-Dimethylamino-isoindol-1-on,
- 7-Ethylamino-pyrrolo[3,4-b]pyridin-5-on,
- 7-Amino-pyrrolo[3,4-b]pyridin-5-on,
- 3-Amino-pyrrolo[3,4-c]pyridin-5-on,
- 3-Amino-6-methyl-pyrrolo[3,4-c]pyridin-1-on,
- 5-Amino-pyrrolo[3,4-b]pyridin-7-on,
- 7-Amino-pyrrolo[3,4-b]pyrazin-5-on,
- 7-Amino-2-methyl-pyrrolo[3,4-b]pyrazin-5-on,
- 7-Amino-2,3-dimethyl-pyrrolo[3,4-b]pyrazin-5-on,
- 7-Amino-2,3-dihydro-[1,4]dithiino[2,3-c]pyrrol-5-on,
- 3-Imino-2-methyl-2,3-dihydro-isoindol-1-on,
- 3-Imino-2-ethyl-2,3-dihydro-isoindol-1-on,
- 3-Imino-2-propyl-2,3-dihydro-isoindol-1-on,
- 2-Hydroxymethyl-3-imino-2,3-dihydro-isoindol-1-on,
- 2-(2-Hydroxyethyl)-3-imino-2,3-dihydro-isoindol-1-on,
- 2-(1-Imino-3-oxo-1,3-dihydro-isoindol-2-yl)-ethansulfonsäure,
- 3-(1-Imino-3-oxo-1,3-dihydro-isoindol-2-yl)-propionsäure,
- 2-(3-Hydroxypropyl)-3-imino-2,3-dihydro-isoindol-1-on,
- 5-Imino-6-methyl-5,6-dihydro-pyrrol[3,4-b]pyridin-7-on
und den kosmetisch akzeptablen Salzen dieser Verbindungen.

3. Verwendung nach Anspruch 1 oder 2, dadurch gekennzeichnet, daß es sich bei den Keratinfasern um menschliche Keratinfasern handelt.

4. Verwendung nach Anspruch 3, dadurch gekennzeichnet, daß es sich um Haar handelt.

5. Zusammensetzung zum Färben von Keratinfasern und insbesondere menschlichen Keratinfasern, wie dem Haar, dadurch gekennzeichnet, daß sie in einem zum Färben geeigneten Medium (i) mindestens eine in den Ansprüchen 1 oder 2 definierte Verbindung der Formel (I) und (ii) eine Verbindung mit primärer oder sekundärer Aminogruppe enthält.

6. Zusammensetzung zum Färben nach Anspruch 5, dadurch gekennzeichnet, daß die Verbindung mit primärer oder sekundärer Aminogruppe eine Verbindung vom aromatischen oder heterocyclischen Typ ist.

7. Zusammensetzung zum Färben nach Anspruch 5, dadurch gekennzeichnet, daß die Verbindung mit primärer oder sekundärer Aminogruppe eine Aminosäure oder ein Oligopeptid ist, das 2 bis 9 Aminosäuren aufweist.

8. Zusammensetzung zum Färben nach einem der Ansprüche 5 bis 7, dadurch gekennzeichnet, daß sie einen pH-Wert im Bereich von 2 bis 11 aufweist.

9. Zusammensetzung nach einem der Ansprüche 5 bis 8, dadurch gekennzeichnet, daß die Verbindung der Formel (I) in einer Konzentration im Bereich von 0,01 bis 5 Gew.-%, bezogen auf das Gesamtgewicht der Zusammensetzung, vorliegt.

10. Zusammensetzung nach einem der Ansprüche 5 bis 9, dadurch gekennzeichnet, daß die Verbindung mit primärer oder sekundärer Aminogruppe in einer Konzentration im Bereich von 0,01 bis 5 Gew.-%, bezogen auf das Gesamtgewicht der Zusammensetzung, vorliegt.

11. Zusammensetzung nach einem der Ansprüche 5 bis 10, dadurch gekennzeichnet, daß das zum Färben geeignete Medium ein wäßriges Medium ist, das aus Wasser und/oder organischen Lösungsmitteln besteht, die unter Alkoholen, Glykolen und Glykolethern in Mengenanteilen im Bereich von 0,5 bis 20 Gew.-%, bezogen auf das Gesamtgewicht der Zusammensetzung, ausgewählt sind.

12. Zusammensetzung zum Färben mit zwei Komponenten, wobei eine Komponente in einem zum Färben geeigneten Medium mindestens eine in den Ansprüchen 1 und 2 definierte Verbindung der Formel (I) und die andere Komponente in einem zum Färben geeigneten Medium eine Verbindung mit primärer oder sekundärer Aminogruppe enthält und die beiden Komponenten, die getrennt voneinander aufbewahrt werden, (i) kurz vor dem Auftragen auf die Keratinfasern vermischt oder (ii) getrennt voneinander auf die Fasern aufgetragen werden.

13. Verfahren zum Färben von Keratinfasern und insbesondere menschlichen Keratinfasern, wie dem Haar, dadurch gekennzeichnet, daß eine Färbemittelzusammensetzung nach einem der Ansprüche 5 bis 12 auf die trockenen oder feuchten Keratinfasern aufgetragen wird, worauf die Zusammensetzung etwa 3 bis 60 min einwirken gelassen wird und anschließend die Fasern gespült, gegebenenfalls gewaschen, von neuem gespült und dann getrocknet werden.

14. Vorrichtung mit mehreren Abteilungen oder "Kits" zum Färben von Keratinfasern, dadurch gekennzeichnet, daß sie mindestens zwei Abteilungen aufweist, wobei eine Abteilung eine Zusammensetzung enthält, die in einem zum Färben geeigneten Medium mindestens eine in den Ansprüchen 1 und 2 definierte Verbindung der Formel (I) enthält, und die andere Abteilung eine Zusammensetzung enthält, die in einem zum Färben geeigneten Medium eine Verbindung mit primärer oder sekundärer Aminogruppe nach einem der Ansprüche 1, 5 bis 7 enthält.
